# EUROPEAN PATENT SPECIFICATION

(11) **EP 2 007 367 B1**
(45) Date of publication and mention of the grant of the patent: **22.12.2010**
(21) Application number: 07751014.7
(22) Date of filing: 16.02.2007
(51) Int. Cl.: A61K 31/065, A61K 9/16

(54) **MEDICATED FEED PREMIX COMPRISING CHLORTETRACYCLINE HYDROCHLORIDE**
MEDIKAMENTENHALTIGER FUTTERPREMIX MIT CHLORTETRACYCLIN-HYDROCHLORID
PRÉ-MÉLANGE ALIMENTAIRE MÉDICAMENTEUX COMPRENANT DU CHLORHYDRATE DE CHLORE TÉTRACYCLINE

(43) Date of publication of application: 31.12.2008
(73) Proprietor: Alpharma, LLC, Fort Lee, NJ 07024 (US)
(72) Inventor: CORNEZ, Robert, B-2950 Kapellen (BE); HOLMES, Steven, Tinleypark, IL 60411 (US); VAN MALCOT, Dirk, B-3111 Wezemaal (BE)
(74) Representative: Sahans, Sarah Anne
(86) International application number: PCT/US2007/004221
(87) International publication number: WO 2008/100262

(56) References cited:
- EP-A1- 1 243 258
- US-A- 2 962 378
- US-A- 3 121 634
- US-A- 3 157 512
- US-A- 3 531 568
- US-A- 4 081 527
- US-B1- 6 773 717

## Description

### BACKGROUND

Antibiotics, such as tetracyclines, are used as growth promoters and feed efficiency promoters in animals such as poultry and livestock, and for therapeutic and prophylactic disease control in animals such as poultry and livestock, fish, domesticated pets, and so forth. Such antibiotics are typically formulated in a medicated animal feed premix or animal feed supplement containing the antibiotic and an edible carrier or diluent. These premixes or animal feed supplements may then be mixed with a sufficient quantity of an appropriate animal foodstuff (e.g., livestock, poultry, fish, pet and/or other animal feed) to provide a final animal feed formulation having the desired level of antibiotic in the feed.

Typical prior art chlortetracycline-containing animal feed premixes are prepared through normal fermentation and dehydration of the mycelium meal, which is added directly to the feed premix. *Streptomyces aureofaciens* is a microbial source organism for chlortetracycline. Under a normal fermentation and dehydration processes, feed premixes comprising about 20 wt% or less of chlortetracycline have been produced. U.S. Patent Number 6,844,006 describes animal feed premixes containing greater than 35 wt% chlortetracycline produced using a novel fermentation process in which the chlortetracycline is also added directly from a fermentation product.

US 6 773 717 B1 discloses chlortetracycline-containing animal feed compositions and processes for their preparation. The composition may be produced in form of granules and may contain binders and carriers. However it teaches the provision of animal feed compositions comprising chlortetracycline in the free base form in order to improve its stability.

US 2 962 378 A discloses the supplementation of a diet with chlortetracycline hydrochloride.

US 3 531 568 A discloses a drinking composition comprising a combination of a tetracycline antibiotic, particularly chlortetracycline hydrochloride, with sulfate ions in order to potentiate the absorption of the antibiotic.

Another problem with prior tetracycline (e.g, chlortetracycline) animal feed premixes is the stability of the tetracycline in these premixes. Because tetracyclines are water soluble, there is intrinsically an issue with stability in finished feeds containing moistures of 10% or higher. The higher water activity (relative vapor pressure) of feedstuff compared to tetracycline leads to an inevitable increase of the humidity of most formulations.

There thus remains a need for alternative animal feed premixes comprising tetracyclines.

### BRIEF SUMMARY

The above-described and other drawbacks are alleviated by an a medicated feed premix composition comprising 15 wt% to 65 wt% of a pharmaceutically acceptable chlortetracycline hydrochloride, 20 wt% to 85 wt% of a carrier, and 0.1 wt% to 10 wt% of a binder, wherein all amounts are based on the total weight of the medicated feed premix. Also included is an animal feed composition comprising an effective amount of the medicated feed premix.

In one embodiment, included is the use of a chlortetracycline hydrochloride in the manufacture of a medicament for treating microbial infections in animals. The medicament comprises 15 wt% to 65 wt% of a pharmaceutically acceptable chlortetracycline hydrochloride, 20 wt% to 85 wt% of a carrier, and 0.1 wt% to 10 wt% of a binder, wherein all amounts are based on the total weight of the medicament.

In another embodiment, a method of making an a medicated feed premix comprises forming a mixture comprising 15 wt% to 65 wt% of a pharmaceutically acceptable chlortetracycline hydrochloride, 20 wt% to 85 wt% of a carrier, 0.1 wt% to 10 wt% of a binder, wherein all amounts are based on the total weight of the medicated feed premix; and granulating the mixture to form a granulate.

In yet another embodiment, a medicated feed premix comprises 15 wt% to 65 wt% of a pharmaceutically acceptable tetracycline hydrochloride, 20 wt% to 85 wt% of a carrier, and 0.1 wt% to 10 wt% of a binder, wherein all amounts are based on the total weight of the animal feed supplement, and wherein the medicated feed premix is in the form of a granulate and has a pH of 3 to 4.

The above-described and other features will be appreciated and understood by those skilled in the art from the following detailed description and appended claims.

### BRIEF DESCRIPTION OF THE DRAWINGS

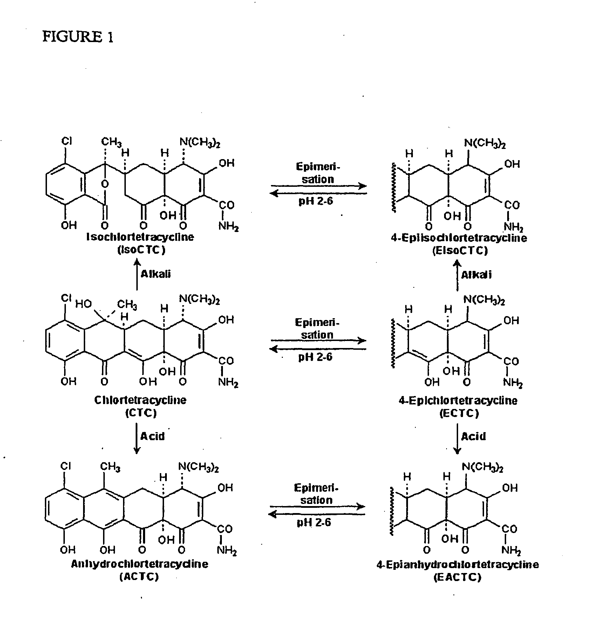

Figure 1 illustrates degradation routes of chlortetracycline.

### DETAILED DESCRIPTION

Disclosed herein are medicated feed premixes comprising pharmaceutically acceptable tetracycline (e.g., chlortetracycline hydrochloride), a binder, and a carrier. A "medicated feed premix" refers to a composition suitable for incorporation into the diet of an animal through incorporation into the animal's food and/or water. In one embodiment, the medicated feed premix is in the form of a granulate. Medicated feed premixes are also referred to as feed "supplement" compositions and "feed additive" compositions.

As used herein, the term tetracycline includes tetracycline, chlortetracycline, oxytetracycline, doxycycline, demeclocycline, lymecycline, meclocycline, methacycline, minocycline, rolitetracycline, and combinations comprising one or more of the foregoing tetracyclines. Included in the term tetracycline are salts, esters, ethers, isomers, mixtures of isomers, complexes or derivatives of the free compound and combinations comprising one or more of the foregoing tetracyclines. In one embodiment, the tetracycline is chlortetracycline hydrochloride.

Since chlortetracycline hydrochloride is the reference standard for chlortetracycline feed grade materials, all chlortetracycline concentrations and percentages stated herein, unless indicated otherwise, are calculated as the hydrochloride equivalent, regardless of the form present (e.g., the free base, complexes or salts other than the hydrochloride, etc.)

Directive 2001/82EC of the European Parliament and of the Council of 6 November 2001 on the Community code relating to veterinary medicinal products-Annex 1. C, describes control of starting materials. In particular, section 1.1 applies to starting materials listed in pharmacopoeias, stating in part "The monographs of the European Pharmacopoeia (EP) shall be applicable to all substances appearing in it." The term "pharmaceutically acceptable" as used herein means a tetracycline ( e.g., chlortetracycline hydrochloride) as listed in the European Pharmacopia, such as the 5^{th} edition of the European Pharmacopia. In one embodiment, pharmaceutically acceptable chlortetracycline hydrochloride comprises a minimum of 89.5 wt% anhydrous chlortetracycline hydrochloride and a maximum of 8 wt% anhydrous tetracycline, with a 94.5-102 wt% sum of anhydrous chlortetracycline hydrochloride plus anhydrous tetracycline hydrochloride.

The medicated feed premixes described herein can be distinguished from prior art tetracycline medicated feed premixes in that the medicated feed premixes described herein comprise pharmaceutically acceptable tetracycline. Pharmaceutically acceptable chlortetracycline hydrochloride, for example, is typically prepared by chemical purification of chlortetracycline hydrochloride produced by fermentation, typically of *Streptomyces aureofaciens.* Prior art chlortetracycline medicated feed premixes contain chlortetracycline hydrochloride in the form of a crude fermentation product such as that produced by dehydration of the mycelium meal. Thus, pharmaceutically acceptable chlortetracycline is produced by chemical purification. In one embodiment, the chlortetracycline hydrochloride is not produced from genetically modified organisms.

The pharmaceutically acceptable tetracycline (e.g., chlortetracycline hydrochloride) comprises 15 wt% to 65 wt% of the total weight of the medicated feed premix, specifically 20 wt% to 65 wt% of the total weight of the medicated feed premix, more specifically 25 wt% to 65 wt% of the medicated feed premix, and most specifically 25 wt% to 60 wt% of the medicated feed premix.

The medicated feed premix also comprises a binder. The term "binder" is well known to those of skill in the art as an agent that holds the components of the formulation together. Suitable binders include, for example, sodium carboxymethylcellulose, hydroxypropylmethylcellulose, gelatin, polyvinylpyrrolidone (PVP), polyvinylalcohol, polyvinylether, hydroxypropylcellulose, potassium alginate, sodium alginate, ethyl cellulose, methylcellulose, microcrystalline cellulose, starch such as partially pregelatinized corn starch, and combinations comprising one or more of the foregoing binders. In one embodiment, the binder comprises sodium carboxymethylcellulose.

The binder comprises 0.1 wt% to 10 wt% of the total weight of the medicated feed premix, specifically 0.2 wt% to 5 wt% of the medicated feed premix.

The medicated feed premix also comprises a carrier. Suitable carriers include, for example, starch, sucrose, glucose, methyl cellulose, magnesium stearate, dicalcium phosphate, calcium sulfate, mannitol, sorbitol, calcium carbonate, and combinations comprising one or more of the foregoing carriers. In one embodiment, the carrier comprises calcium sulfate.

The carrier comprises 20 wt% to 85 wt% of the total weight of the medicated feed premix, specifically 30 wt% to 75 wt% of the medicated feed premix.

The medicated feed premix optionally comprises a pH regulator. In some embodiments, the carrier (e.g., calcium sulfate) is sufficient to maintain the pH of the composition, although it is possible to add an additional pH regulator. Suitable pH regulators include acids and bases suitable to adjust the pH to the desired level. In one embodiment, the pH regulator comprises a base such as an alkali metal or alkaline earth metal hydroxide, including sodium hydroxide, potassium hydroxide, calcium hydroxide, and combinations comprising one or more of the foregoing pH regulators.

The pH regulator comprises 0 wt% to 5 wt% of the total weight of the medicated feed premix, specifically 0.1 wt% to 2 wt% of the medicated feed premix.

The medical feed premix may comprise additional additives such as fillers, lubricants, disintegrants, and combinations thereof.

In one embodiment, the pH of the medicated feed premix is between 3 and 4 in order to prevent significant degradation towards degradants with know toxicity profiles such as the anhydro or iso forms of tetracycline. It was unexpected that such a low pH can provide a chlortetracycline medicated feed premix having exceptional stability.

The pharmaceutically acceptable tetracycline, carrier, binder and optional pH regulator are combined to form the medicated feed premix. In one embodiment, the medicated feed premix is a multiparticulate composition. The term multiparticulate is intended to refer broadly to small particles regardless of their composition or the manner in which they are formed. The particles generally are of a mean diameter of about 40 to about 3000 µm, specifically about 30 to about 1000 µm.

In one embodiment, the tetracycline, carrier and binder are granulated to form the medicated feed premix. Advantages of a granular formulation include: higher stability due to the physical seclusion of the tetracycline from the environment; higher stability due to the chemical protection by the carrier; better mixability due to the match in particle size of the feed and the product; less risk for carry over or cross contamination to other feeds produced in the same plant; less health risks for the operators handling the product; and better mechanical properties such as flowability.

Granulation is a process by which relatively small particles are built up into larger granular particles. In wet-granulation, a liquid is used to increase the intermolecular forces between particles, leading to an enhancement in granular integrity, referred to as the "strength" of the granule. Examples of liquids found to be effective wet-granulation liquids include water, ethanol, isopropyl alcohol and acetone. In one embodiment, the wet-granulation liquid is water at pH 7 or higher.

In an exemplary granulation process, the components of the medicated feed premix are formed into a mixture and the mixture is granulated to form the granulate.

Several types of wet-granulation processes can be used to form tetracycline-containing multiparticulates. Examples include fluidized bed granulation, rotary granulation and high-shear mixers. In fluidized bed granulation, air is used to agitate or "fluidize" particles of chlortetracycline and/or carrier in a fluidizing chamber. The liquid is then sprayed into this fluidized bed, forming the granules. In rotary granulation, horizontal discs rotate at high speed, forming a rotating "rope" oftetracycline and/or carrier particles at the walls of the granulation vessel. The liquid is sprayed into this rope, forming the granules. High-shear mixers contain an agitator or impeller to mix the particles of chlortetracycline and/or carrier. The liquid is sprayed into the moving bed of particles, forming granules. In these processes, the liquid preferably comprises the binder and optional pH regulator. Also in these processes, all or a portion of the carrier can be dissolved into the liquid prior to spraying the liquid onto the particles. Thus, in these processes, the steps of forming the liquid mixture and forming particles from the liquid mixture occur simultaneously.

In another embodiment, the particles are formed by extruding the liquid mixture into a solid mass followed by spheronizing or milling the mass. In this process, the liquid mixture, which is in the form of a paste-like plastic suspension, is extruded through a perforated plate or die to form a solid mass, often in the form of elongated, solid rods. This solid mass is then milled to form the multiparticulates. In one embodiment, the solid mass is placed, with or without an intervening drying step, onto a rotating disk that has protrusions that break the material into multiparticulate spheres, spheroids, or rounded rods. The so-formed multiparticulates are then dried to remove any remaining liquid. This process is sometimes referred to in the pharmaceutical arts as an extrusion/spheronization process.

Once the particles are formed, a portion of the liquid is removed, typically in a drying step, thus forming the multiparticulates. Preferably, at least 80% of the liquid is removed from the particles, more preferably at least 90%, and most preferably at least 95% of the liquid is removed from the particle during the drying step.

The multiparticulates may also be made by a granulation process comprising the steps of (a) forming a solid mixture comprising tetracycline, a carrier, a binder, and optionally a pH regulator; and (b) granulating the solid mixture to form multiparticulates. Examples of such granulation processes include dry granulation and melt granulation, both well known in the art.

An example of a dry granulation process is roller compaction. In roller compaction processes, the solid mixture is compressed between rollers. The rollers can be designed such that the resulting compressed material is in the form of small beads or pellets of the desired diameter. Alternatively, the compressed material is in the form of a ribbon that may be milled to for multiparticulates using methods well known in the art.

In a melt granulation processes, the solid mixture is fed to a granulator that has the capability of heating or melting the carrier. Equipment suitable for use in this process includes high-shear granulators and single or multiple screw extruders, such as those described above for melt-congeal processes. In melt granulation processes, the solid mixture is placed into the granulator and heated until the solid mixture agglomerates. The solid mixture is then kneaded or mixed until the desired particle size is attained. The so-formed granules are then cooled, removed from the granulator and sieved to the desired size fraction, thus forming the multiparticulates.

In one embodiment, the medicated feed premixes are blended with an animal foodstuff to produce a medicated finished complete or supplement feed product. Animals to which the medicated feed premixes may be administered include pets or companion animals, and ranch or farm animals or other livestock, such as animals raised as a food source or for another commercial purpose. Such animals include, but are not limited to, cattle, sheep, horses, pigs, buffalo, goats, dogs, cats, rabbits, rats, mice, minks, fish, and fowl (including egg-laying or edible fowl, such as chickens, turkeys, geese, ducks, quail, pheasant, etc.), and the like.

In one embodiment, a medicated animal feed composition is prepared by adding a medicated feed premix to an animal feedstuff. The medicated feed premix may be added to the feedstuff in a number of ways. The medicated feed premix containing a given quantity of chlortetracycline may be added to a given quantity of feed and mixed or blended to provide a substantially homogeneous medicated feed composition. Large feed lots may be prepared in this manner for treating a large number of animals. Alternatively, feed batches containing feed for a single animal or single meal may be prepared either by mixing a predetermined quantity of medicated feed premix with the animal feed or by adding a predetermined quantity of premix to an animal's feed as a top dressing.

Manufactured foodstuffs for animals such as cattle, pigs, and fowl are usually provided in the form of pellets or similar particulate material. Pellets are typically manufactured by combining a cereal base with ingredients such as oil and protein, steam conditioning the mixture (for example at 70°C for 5 minutes), extruding through a circular die (typically between 2 mm and 15 mm in diameter), cutting into appropriately sized lengths (e.g., 5-20 mm), and drying. The finished pellets are generally cylindrically shaped and have a relatively smooth surface.

By varying the quantity of medicated feed premix added to the animal feedstuff, the concentration of the active ingredient in the final medicated feed mixture may be adjusted to meet particular needs and may be varied over a wide range. An effective amount of the medicated feed premix is added to the feedstuff. The minimum concentration should be such as to achieve the desired result (therapeutic, prophylactic, growth enhancement, etc.). The maximum concentration should be such as to avoid any undesirable side effects when the feed rations are ingested by the animal. Within these limitations, specific amounts of active ingredient will normally be regulated by the practitioner according to potency of the medicated feed premix and the usual recommended dosing levels for the active ingredient. The practitioner will generally take into account a number of factors, such as the animal species being treated, the animal's age or stage of development, the frequency of administration, whether the composition is being administered therapeutically or prophylactically and the degree of antimicrobial results sought, the severity of any disease being treated, and so forth. The selection of dosages and dosage regimens is generally performed as a routine matter by persons skilled in the arts pertaining to veterinary medicine, animal husbandry and nutrition, and the like. Accordingly, the indications and dosage levels given herein are intended to be exemplary and illustrative only, and are not intended to be exhaustive or restrictive of the present invention as claimed.

The medicated feed premix may in this case be mixed with feedstuffs or with drinking water. The tetracycline concentrations in feedstuffs or in drinking water may vary within certain limits. The chlortetracycline concentrations are in general between 5 and 2000 ppm, specifically between 10 and 1000 ppm, and more specifically between 10 and 500 ppm in the feedstuff or drinking water.

The treatment method can also be extended to other methods for treating and feeding animals. Thus, for example, the medicated feed premix may be combined with other active substances, such as, for example, with other anti-coccidial agents.

In one embodiment, a method of combating microbial infection in animals such as pigs, wherein the medicated feed premixes are administered orally ad lib, and in particular, either as feedstuff or by means of drinking water.

In another embodiment, included are methods of improving the stability of tetracycline medicated feed premixes. Tetracyclines degrade, for example, by reversible epimerization to the less active 4-epitetracycline. The degree of epimerization is dependent upon pH, and is greatest at a pH of approximately 3. The rate of epimerization is also affected by temperature and the presence of phosphate or citrate ions. Other degradation routes include anhydration and isomerization which typically become important outside of the pH 2.5 to 5 range. Figure 1 illustrates various degradation routes of tetracycline.

Unexpectedly, it has been discovered that the stability of the tetracycline medicated feed premix is improved by adjusting the pH of the premix to 3 to 4. At pH 3 to 4, the premix is in a range where tetracycline degradation is a high risk, but with a predominant route which evades degradation to potentially toxic components.

The invention is further illustrated by the following non-limiting examples.

### EXAMPLES

### Preparation of 25% chlortetracycline medicated feed premix

Step no. 1: Weighing of the starting materials: The 25% chlortetracycline animal feed supplement is formulated by using chlortetracycline hydrochloride, calcium sulfate dihydrate and sodium carboxymethylcellulose.

Step no. 2: Mixing: The weighed starting materials are unloaded into a horizontal ribbon blender. The nominal load is 1 metric ton. The blender is positioned on load cells, and the actual weight is measured and recorded.

The sequence of adding the starting materials to the mixer is as follows:
Calcium sulfate dihydrate: 40% of batch size
Chlortetracycline hydrochloride: 25% (w/w) of batch size
Sodium carboxymethylcellulose: 3% of batch size
Calcium sulfate dihydrate: up to 100% of batch size

The mixing time is 20 minutes at a mixing speed of 16 rpm.

Step no. 3: Granulation: After blending, the powder mix is transferred to a hopper. The hopper gradually feeds the "High Shear Granulation" (HSG) unit. The granulator processes a sequential relatively small amount of material (approximately 3 kg). The HSG-unit consists of two parts. The first part is the wetting phase. Water, of potable quality, is metered into the granulator by means of a dosing pump. The second part is the granulation phase, where wet granules are formed. The parameters for granulation are automatically controlled by PLC (Programmable Logic Controller).

Step no. 4: Drying: Afterwards, the wet granules are transferred to a fluidized bed dryer (hot air dryer). The temperature of the product in the fluid bed dryer is maintained between 35°C and 50°C. The parameters for drying are automatically controlled by PLC (Programmable Logic Controller).

Step no. 5: Sieving: After drying step, the dry granules are sieved through a vibrating screen, in order to achieve the specified particle size. Oversized granules (> 1180 µm) are milled, and are collected together with the fines (< 300 µm) in a hopper. This hopper feeds the high shear granulator. The re-granulated product is dried and sieved again.

Step no. 6: Homogenization: The product (in range with the particle size) leaves the vibrating screen and is collected in a hopper until the end of the granulation production. Prior to packaging the granules are homogenized in a second mixer for 5 minutes.

Step no. 7: Packaging: The final step after sieving at the end of the manufacturing process is the packing of the finished product in polyethylene bags. From the second mixer, the granules are moved over the funnel to the packaging unit.

### Results

Three batches of 25% chlortetracycline medicated feed premixes were compared for shelf stability stored in paper and plastic. Table 1 shows the stability of different batches of medicated feed premixes stored in paper or plastic as the percentage of chlortetracycline remaining in the formulation. Time points were 1 month (T1M), 3 months (T3M), 9 months (T9M), 12 months (T12M), eighteen months (T18M), and 24 months (T24M). RH denotes the relative humidity in the storage conditions.

**Table 1: Overview Stability 25% Chlortetracycline feed premixes**

| Primary packaging | Storage condition | Time point | Batch | | |
|---|---|---|---|---|---|
| | | | Batch 1 | Batch 2 | Batch 3 |
| Paper | Initial | Initial | 97.0 | 98.6 | 95.8 |
| | 25°C/60% RH | T1M | 99.0 | 98.7 | 97.8 |
| | | T3M | 98.2 | ⁽³⁾ | 101.6 |
| | | T9M | 98.6 / 95.5 | 99.6 / 96.6 | 99.3 / 95.5 |
| | | T12M | 98.4 / 94.8 | 98.6 / 96.1 | 98.4 / 95.8 |
| | | T18M | 94.8 | 96.2 | 95.6 |
| | | T24M | 95.4 | 96.1 | 95.3 |
| | 40°C/75% RH | T1M | 94.3 | 97.9 | 96.4 |
| | | T3M | 97.2 | 97.4 | 98.4 |
| | | T13.5M | 92.6 | 93.0 | 94.0 |
| | 30°C/60% RH | T18M | 94.4 | 94.6 | 94.5 |
| | | T24M | 95.1 | 95.0 | 95.1 |
| Plastic | 25°C/60% RH | T1M | - | - | - |
| | | T3M | 98.0 | 100.6 | 102.5 |
| | | T9M | 95.4 | 97.5 | 95.8 |
| | | T12M | 99.3 / 95.0 | 100.7 / 96.8 | 100.0 / 95.4 |
| | | T18M | 94.5 | 95.1 | 95.7 |
| | | T24M | 95.9 | 96.8 | 96.1 |
| | 40°C/75% RH | T1M | - | - | - |
| | | T3M | 97.1 | 96.0 | 99.2 |
| | | T13.5M | 92.8 | 92.6 | 93.7 |
| | 30°C/60% RH | T18M | 93.5 | 93.5 | 95.5 |
| | | T24M | 93.8 | 95.1 | 95.4 |

| | | | | | |
|---|---|---|---|---|---|
| -: No analysis required according to protocol. | | | | | |

Both 10% and 25% chlortetracycline hydrochloride feed premixes were produced and then mixed into either a commercial mash feed or a commercial pellet feed composition at 500 ppm with a planetary mixer. The amount of chlortetracycline was measured by HPLC analysis.

**Table 2: Comparative example- Summary results on the recovery and stability of chlortetracycline hydrochloride in pig mash and pelleted feeds supplemented with 10% chlortetracycline feed premix**

| Lot code | Type of product | Mean recovery in % at different time points of storage in months | | | |
|---|---|---|---|---|---|
| | | T = 0 | T = 0.5 | T = 1 | T=2 |
| 10% lot 1 | Mash feed | 93.9 | 111.1 | 101.0 | 114.0 |
| 10% lot 2 | Mash feed | 95.3 | 102.0 | 104.0 | 110.9 |
| 10% lot 3 | Mash feed | 99.3 | 96.1 | 110.9 | 107.4 |
| | | | | | |
| 10% lot 1 | Mash feed (Sp)* | 93.9 | 108.8 | 112.5 | 105.6 |
| 10% lot 2 | Mash feed (Sp) | 95.3 | 101.6 | 114.5 | 116.2 |
| 10% lot 3 | Mash feed (Sp) | 99.3 | 115.5 | 92.9 | 100.0 |
| | | | | | |
| 10% lot 1 | Pellet feed | 86.8 | 79.5 | 73.4 | 68.0 |
| 10% lot 2 | Pellet feed | 93.0 | 85.8 | 81.6 | 75.0 |
| 10% lot 1 | Pellet feed | 91.1 | 80.5 | 76.7 | 66.2 |

**Table 3: Summary results on the recovery and stability of chlortetracycline hydrochloride in pig mash and pelleted feeds supplemented with 25% Chlortetracycline feed premix**

| Lot code | Feed | Time points of storage at 25°C and 60 % RH in months | | | | |
|---|---|---|---|---|---|---|
| | | 0 | 0.5 | 1 | 2 | 3 |
| | | | | | | |
| 25% lot 1 | Mash feed | 95.2 | 101.6 | 97.9 | 101.0 | 106.0 |
| 25% lot 2 | Mash feed | 100.4 | 106.0 | 100.8 | 92.0 | 102.5 |
| 25% lot 3 | Mash feed | 111.3 | 98.5 | 110.1 | 96.7 | 106.9 |
| | | | | | | |
| Mean mash feeds | | 102.3 | 102.1 | 103.0 | 96.6 | 105.1 |
| | | | | | | |
| 25% lot 1 | Pellet feed | 85.6 | 87.9 | 84.9 | 87.1 | 74.2 |
| 25% lot 2 | Pellet feed | 88.9 | 89.8 | 88.0 | 82.1 | 75.3 |
| 25% lot 3 | Pellet feed | 102.4 | 89.6 | 99.6 | 83.1 | 79.9 |
| | | | | | | |
| Mean pelleted feeds | | 92.3 | 89.1 | 90.8 | 84.1 | 76.5 |

Tables 2 and 3 summarize the stability of the 10% chlortetracycline feed premix compared to 25% in different types of feed: Mash and pellets. The 10% and 25% chlortetracycline hydrochloride premixes perform well in mash feed, but differ in pellets. Where the 10% fails completely at T2M (failing is a percentage below 75%), acceptable results at T3M for the 25% supplement are observed.

Disclosed herein are chlortetracycline animal feed compositions comprising pharmaceutically acceptable chlortetracycline hydrochloride. The compositions have the advantage of compliance with EU regulations as given in Directive 2001/82EC of the European Parliament and of the Council of 6 November 2001 on the Community code relating to veterinary medicinal products-Annex 1. C. Other advantages include shelf stability, stability in feed, safety for both the animal and the consumer, and improved availability to the animal. In addition, use of a granular formulation provides both user convenience and consumer safety.

## Claims

1. A medicated feed premix, comprising:
15 wt% to 65 wt% of a pharmaceutically acceptable chlortetracycline hydrochloride,
20 wt% to 85 wt% of a carrier, and
0.1 wt% to 10 wt% of a binder,
wherein all amounts are based on the total weight of the medicated feed premix.

2. The medicated feed premix of claim 1, wherein the binder comprises sodium carboxymethylcellulose.

3. The medicated feed premix of claim 1, wherein the carrier comprises calcium sulfate.

4. The medicated feed premix of claim 1, having a pH of 3 to 4.

5. The medicated feed premix of claim 1, comprising 20 wt% to 65 wt% of the pharmaceutically acceptable chlortetracycline hydrochloride.

6. The medicated feed premix of claim 1, wherein the pharmaceutically acceptable chlortetracycline hydrochloride comprises a minimum of 89.5 wt% anhydrous chlortetracycline hydrochloride and a maximum of 8 wt% anhydrous tetracycline, with a 94.5-102 wt% sum of anhydrous chlortetracycline hydrochloride plus anhydrous tetracycline hydrochloride.

7. The medicated feed premix of claim 1, in the form of a multiparticulate composition.

8. The medicated feed premix of claim 7, wherein the multiparticulate composition is a granulate.

9. The use of a chlortetracycline hydrochloride in the manufacture of a medicament for treating microbial infections in animals, wherein the medicament comprises:
20 wt% to 65 wt% of a pharmaceutically acceptable chlortetracycline hydrochloride,
20 wt% to 85 wt% of a carrier, and
0.1 wt% to 10 wt% of a binder,
wherein all amounts are based on the total weight of the medicament.

10. The use of claim 9, wherein the medicament is administered either as a feedstuff or by means of drinking water.

11. The use of claim 9, wherein the carrier comprises calcium sulfate.

12. The use of claim 9, wherein the medicated feed premix has a pH of 3 to 4.

13. An animal feed composition comprising:
an animal foodstuff, and
an effective amount of a medicated feed premix comprising:
15 wt% to 65 wt% of a pharmaceutically acceptable chlortetracycline hydrochloride,
20 wt% to 85 wt% of a carrier, and
0.1 wt% to 10 wt% of a binder,
wherein all amounts are based on the total weight of the medicated feed premix.

14. The animal feed composition of claim 13, wherein the carrier comprises calcium sulfate.

15. The animal feed composition of claim 13, wherein the medicated feed premix has a pH of 3 to 4.

## Patentansprüche

1. Ein medikamentenhaltiger Futterpremix, welcher umfasst:
15 Gew.-% bis 65 Gew.-% eines pharmazeutisch verträglichen Chlortetracyclin-Hydrochlorids,
20 Gew.-% bis 85 Gew.-% eines Trägers und
0,1 Gew.-% bis 10 Gew.-% eines Bindemittels,
wobei alle Mengen auf das Gesamtgewicht des medikamentenhaltigen Futterpremixes bezogen sind.

2. Der medikamentenhaltige Futterpremix nach Anspruch 1, wobei das Bindemittel Natriumcarboxymethylcellulose umfasst.

3. Der medikamentenhaltige Futterpremix nach Anspruch 1, wobei der Träger Calciumsulfat umfasst.

4. Der medikamentenhaltige Futterpremix nach Anspruch 1, welcher einen pH von 3 bis 4 aufweist.

5. Der medikamentenhaltige Futterpremix nach Anspruch 1, welcher 20 Gew.-% bis 65 Gew.-% des pharmazeutisch verträglichen Chlortetracyclin-Hydrochlorids umfasst.

6. Der medikamentenhaltige Futterpremix nach Anspruch 1, wobei das pharmazeutisch verträgliche Chlortetracyclin-Hydrochlorid ein Minimum von 89,5 Gew.-% wasserfreiem Chlortetracyclin-Hydrochlorid und ein Maximum von 8 Gew.-% wasserfreiem Tetracyclin umfasst, wobei die Summe von wasserfreiem Chlortetracyclin-Hydrochlorid plus wasserfreiem Tetracyclin-Hydrochlorid 94,5 - 102 Gew.-% ausmacht.

7. Der medikamentenhaltige Futterpremix nach Anspruch 1 in der Form einer multipartikulären Zusammensetzung.

8. Der medikamentenhaltige Futterpremix nach Anspruch 7, wobei die multipartikuläre Zusammensetzung ein Granulat ist.

9. Die Verwendung eines Chlortetracyclin-Hydrochlorids bei der Herstellung eines Medikaments zur Behandlung mikrobieller Infektionen bei Tieren, wobei das Medikament umfasst:
20 Gew.-% bis 65 Gew.-% eines pharmazeutisch verträglichen Chlortetracyclin-Hydrochlorids,
20 Gew.-% bis 85 Gew.-% eines Trägers und
0,1 Gew.-% bis 10 Gew.-% eines Bindemittels,
wobei alle Mengen auf das Gesamtgewicht des Medikaments bezogen sind.

10. Die Verwendung nach Anspruch 9, wobei das Medikament entweder als ein Futtermittel oder mit Hilfe von Trinkwasser verabreicht wird.

11. Die Verwendung nach Anspruch 9, wobei der Träger Calciumsulfat umfasst.

12. Die Verwendung nach Anspruch 9, wobei der medikamentenhaltige Futterpremix einen pH von 3 bis 4 aufweist.

13. Eine Tierfutterzusammensetzung, welche umfasst:
ein Nahrungsmittel für Tiere und
eine wirksame Menge eines medikamentenhaltigen Futterpremixes, welcher umfasst:
15 Gew.-% bis 65 Gew.-% eines pharmazeutisch verträglichen Chlortetracyclin-Hydrochlorids,
20 Gew.-% bis 85 Gew.-% eines Trägers und
0,1 Gew.-% bis 10 Gew.-% eines Bindemittels,
wobei alle Mengen auf das Gesamtgewicht des medikamentenhaltigen Futterpremixes bezogen sind.

14. Die Tierfutterzusammensetzung nach Anspruch 13, wobei der Träger Calciumsulfat umfasst.

15. Die Tierfutterzusammensetzung nach Anspruch 13, wobei der medikamentenhaltige Futterpremix einen pH von 3 bis 4 aufweist.

## Revendications

1. Prémélange alimentaire médicamenteux comprenant :
15 % en poids à 65 % en poids d'un chlorhydrate de chlorotétracycline acceptable au plan pharmaceutique,
20 % en poids à 85 % en poids d'un support et
0,1 % en poids à 10 % en poids d'un liant,
où toutes les quantités sont basées sur le poids total du prémélange alimentaire médicamenteux.

2. Prémélange alimentaire médicamenteux selon la revendication 1, dans lequel le liant comprend de la carboxyméthylcellulose de sodium.

3. Prémélange alimentaire médicamenteux selon la revendication 1, dans lequel le support comprend du sulfate de calcium.

4. Prémélange alimentaire médicamenteux selon la revendication 1, présentant un pH de 3 à 4.

5. Prémélange alimentaire médicamenteux selon la revendication 1, comprenant 20 % en poids à 65 % en poids du chlorhydrate de chlorotétracycline acceptable au plan pharmaceutique.

6. Prémélange alimentaire médicamenteux selon la revendication 1, dans lequel le chlorhydrate de chlorotétracycline acceptable au plan pharmaceutique comprend un minimum de 89,5 % en poids de chlorhydrate de chlorotétracycline anhydre et un maximum de 8 % en poids de tétracycline anhydre, avec une somme de 94,5 à 102 % en poids de chlorhydrate de chlorotétracycline anhydre et de chlorhydrate de tétracycline anhydre.

7. Prémélange alimentaire médicamenteux selon la revendication 1, préparé sous la forme d'une composition multiparticulaire.

8. Prémélange alimentaire médicamenteux selon la revendication 7, dans lequel la composition multiparticulaire est un granulé.

9. Utilisation d'un chlorhydrate de chlorotétracycline dans la fabrication d'un médicament pour le traitement d'infections microbiennes chez les animaux, le médicament comprenant :
20 % en poids à 65 % en poids d'un chlorhydrate de chlorotétracycline acceptable au plan pharmaceutique,
20 % en poids à 85 % en poids d'un support, et
0,1 % en poids à 10 % en poids d'un liant,
où toutes les quantités sont basées sur le poids total du médicament.

10. Utilisation selon la revendication 9, où le médicament est administré sous la forme d'aliment pour animaux ou au moyen de l'eau de boisson.

11. Utilisation selon la revendication 9, dans laquelle le support comprend du sulfate de calcium.

12. Utilisation selon la revendication 9, dans laquelle le prémélange alimentaire médicamenteux a un pH de 3 à 4.

13. Composition d'aliment pour animaux comprenant :
un produit alimentaire pour animaux, et
une quantité efficace de prémélange alimentaire médicamenteux comprenant :
15 % en poids à 65 % en poids d'un chlorhydrate de chlorotétracycline acceptable au plan pharmaceutique,
20 % en poids à 85 % en poids d'un support, et
0,1 % en poids à 10 % en poids d'un liant,
où toutes les quantités sont basées sur le poids total du prémélange alimentaire médicamenteux.

14. Composition d'aliments pour animaux selon la revendication 13, dans laquelle le support comprend du sulfate de calcium.

15. Composition d'aliments pour animaux selon la revendication 13, dans laquelle le prémélange alimentaire médicamenteux a un pH de 3 à 4.
